# EUROPÄISCHE PATENTSCHRIFT

(11) **EP 3 615 181 B1**
(45) Veröffentlichungstag und Bekanntmachung des Hinweises auf die Patenterteilung: **06.01.2021**
(21) Anmeldenummer: 18725753.0
(22) Anmeldetag: 18.04.2018
(51) Int. Cl.: B01D 53/18, B01D 53/50, B01D 53/56, B01D 53/58, B01D 53/62, B01D 53/68, B01D 53/78, C07C 273/14

(54) **VORRICHTUNG UND VERFAHREN ZUR ABGASWÄSCHE**
APPARATUS AND METHOD FOR WASTE GAS SCRUBBING
DISPOSITIF ET PROCÉDÉ D'ÉPURATION DES EFFLUENTS GAZEUX

(30) Priorität: 25.04.2017 DE 102017108843
(43) Veröffentlichungstag der Anmeldung: 04.03.2020
(73) Patentinhaber: thyssenkrupp Industrial Solutions AG, 45143 Essen (DE); thyssenkrupp AG, 45143 Essen (DE)
(72) Erfinder: GEHRKE, Helmut, 59192 Bergkamen (DE); SCHMITZ, Martina, 45239 Essen (DE); BUSS, Stephan, 44143 Dortmund (DE)
(74) Vertreter: thyssenkrupp Intellectual Property GmbH
(86) Internationale Anmeldenummer: PCT/EP2018/059822
(87) Internationale Veröffentlichungsnummer: WO 2018/197279

(56) Entgegenhaltungen:
- WO-A1-2015/170293
- AT-A1- 506 546
- US-A- 5 403 568
- US-A1- 2011 280 779
- US-A1- 2015 238 897

## Beschreibung

Die Erfindung betrifft eine Harnstoffanlage gemäß Anspruch 1, in die eine Vorrichtung zur Abgaswäsche integriert ist, wobei die Vorrichtung einen Kanal mit einer Eingangsöffnung und einer Ausgangsöffnung umfasst, wobei die Vorrichtung derart ausgestaltet ist, dass ein Abgas entlang einer Transportrichtung (T) im Kanal einen ersten Bereich zur Entfernung von Staubpartikeln aus dem Abgas und einen zweiten Bereich zur Entfernung von chemischen Verbindungen aus dem Abgas, welche durch eine Säure-Base-Reaktion in eine wässrige Flüssigphase integriert werden können, passiert, wobei eine senkrecht zur Transportrichtung (T) verlaufende Querschnittsfläche des Kanals im zweiten Bereich größer ist als eine senkrecht zur Transportrichtung (T) verlaufende Querschnittsfläche im ersten Bereich.

Gegenstand der vorliegenden Erfindung ist ein Verfahren zur Abgaswäsche, welches in einer Harnstoffanlage stattfindet.

Die erfindungsgemäße Vorrichtung ist dazu vorgesehen, Staubpartikel und chemische Verbindungen, welche durch eine Säure-Base-Reaktion in eine wässrige Flüssigphase integriert werden können, abzuscheiden, um zu verhindern, dass behördlich vorgegebene Schwellwerte im von der Vorrichtung ausgestoßenen Abgas überschritten werden.

Hierzu wird das Abgas durch die erfindungsgemäße Vorrichtung geleitet und passiert hierbei typischerweise nacheinander einen ersten Bereich, in dem Staubpartikel aus dem Abgas entfernt werden, und einen zweiten Bereich, in dem die genannten chemischen Verbindungen aus dem Abgas entfernt werden. Dabei wird das Abgas jeweils durch eine Flüssigkeitsdusche geleitet, wobei in einem ersten Schritt die Staubpartikel durch die Flüssigkeitsdusche abgeschieden oder niedergeschlagen werden und in einem zweiten Schritt die genannten chemischen Verbindungen mit mindestens einem Bestandteil der in dieser Stufe verwendeten Flüssigkeitsdusche reagieren und so in die wässrige Flüssigphase übergehen.

Die erfindungsgemäße Vorrichtung ist in einer Harnstoffanlage integriert, d.h. die erfindungsgemäße Vorrichtung ist dazu vorgesehen, in einem ersten Bereich Harnstoffstaubpartikel abzuscheiden oder niederschlagen zu lassen und in einem zweiten Bereich gasförmigen Ammoniak durch Integration in die wässrige Flüssigphase zu entfernen, indem man sich einer Säure bedient, welche in der Waschlösung durch Reaktion Ammonium bildet und dann entsprechend zur Entfernung von Ammoniak führt.

### Horizontale und vertikale Vorrichtungen zur Abgaswäsche sind dem Fachmann bekannt:

US 5,403,568 offenbart eine Vorrichtung zur zweistufigen Entfernung von Schwefeldioxid aus heißen Rauchgasen. Hier handelt es sich um einen so genannten Horizontalwäscher, bei dem das heiße Rauchgas die Vorrichtung in horizontaler Richtung durchströmt, wobei eine sich vertikal erstreckende Sprühvorrichtung mit Sprühdüsen vorgesehen ist, die ein wässriges Waschmedium versprühen, welches in einem Strom parallel zum Strom des Rauchgases versprüht wird. Bei diesem Horizontalwäscher sind mehrere beabstandete Sprühvorrichtungen vorgesehen, die jedoch alle dazu dienen, ein Gas, nämlich Schwefeldioxid aus dem Gasstrom zu entfernen und somit funktionell gleich sind. Dieser Horizontalwäscher hat zudem ein rechteckiges Gehäuse, dessen wirksame Querschnittsfläche in den nachfolgenden Waschvorrichtungen gleich groß ist wie in der ersten Waschvorrichtung. Stromabwärts ist noch eine Vorrichtung vorgesehen, um Flüssigkeit aus dem Gasstrom zu entfernen. Zur Entfernung von Schwefeldioxid wird bei dieser bekannten Vorrichtung eine alkalische Waschlösung verwendet, die Kalk enthält.

Finishing with Emissions, Fertilizer International 469, November/Dezember 2015, Seiten 25-31, offenbart eine Vorrichtung zur mehrstufigen Entfernung von Staubpartikeln und Ammoniak aus Abgasen.

In der DE 10 2012 111 185 A1 wird ein Apparat zur Gaswäsche beschrieben, insbesondere zur Abtrennung von Kohlendioxid aus Rauchgas mittels Aminwäsche. Dieser bekannte Apparat umfasst eine Kühlstufe zur Direktkühlung und zur Vorwäsche eines in den Apparat eintretenden Gasstromes mittels einer der Kühlstufe zugeführten Flüssigkeit. Es wird nicht explizit ausgeführt, dass diese Vorwäsche dazu dient, Staubpartikel aus dem Rauchgas zu entfernen. Anschließend gelangt der Gasstrom in eine Absorptionsstufe, der eine Waschflüssigkeit zugeführt wird. Die Waschstufe enthält Kolonneneinbauten zur Vergrößerung der Austauschfläche für einen Stoffaustausch zwischen dem Gasstrom und der Waschflüssigkeit, wobei die Kolonneneinbauten aus Füllkörpern oder strukturierten Packungen bestehen können. Die Kühlstufe kann als Sprühapparat ausgebildet sein mit einer Mehrzahl von Sprühdüsen. Bei diesem bekannten Apparat handelt es sich um einen Waschturm, bei dem die Kühlstufe und die Absorptionsstufe in einer einzigen Vorrichtung vertikal übereinander angeordnet sind. Das mittels der Gaswäsche zu reinigende Rauchgas durchströmt den Waschturm in vertikaler Richtung.

Die AT 506 546 A1 zeigt eine Vorrichtung zur Abscheidung von Partikeln und chemischen Verbindungen aus Verbrennungsabgasen mit zwei Bereichen unterschiedlichen Strömungsquerschnittes, welcher zumindest teilweise horizontal verläuft.

Die WO 2015/170293 A1 offenbart die Reinigung eines Gasstromes einer Harnstoffanlage, welcher Harnstoffstaub und Ammoniak enthält. Dazu ist eine Vorrichtung zur Abgasreinigung vorgesehen, die zwei zwei elektrostatische Entstauber und umfasst, welche horizontal durchströmt werden.

Die bekannten Vorrichtungen und Verfahren zur Abgaswäsche sind jedoch nicht in jeder Hinsicht zufriedenstellend und es besteht ein Bedarf an verbesserten Vorrichtungen und Verfahren.

Es ist daher eine Aufgabe der Erfindung, Vorrichtungen und Verfahren zur Abgaswäsche bereitzustellen, mit welchen die Effizienz für das Entfernen der Staubpartikel, insbesondere von Harnstoffstaubpartikeln, und der genannten chemischen Verbindungen, insbesondere von Ammoniak, aus dem Abgas gegenüber denen aus dem Stand der Technik verbessert ist.

Diese Aufgabe wird durch den Gegenstand der Patentansprüche und der Beschreibung gelöst.

Ein erster Aspekt der Erfindung betrifft eine Harnstoffanlage nach Anspruch 1, in die eine Vorrichtung (1) zur Abgaswäsche integriert ist, wobei die Vorrichtung einen Kanal (5) mit einer Eingangsöffnung (10) und einer Ausgangsöffnung (20) umfasst, wobei die Vorrichtung (1) derart ausgestaltet ist, dass ein Abgas entlang einer Transportrichtung (T) im Kanal (5)
- einen ersten Bereich (11) zur Entfernung von Staubpartikeln aus dem Abgas und
- einen zweiten Bereich (12) zur Entfernung von chemischen Verbindungen aus dem Abgas, welche durch eine Säure-Base-Reaktion in eine wässrige Flüssigphase integriert werden können, passiert,
wobei eine senkrecht zur Transportrichtung (T) verlaufende Querschnittsfläche des Kanals (5) im zweiten Bereich (12) größer ist als eine senkrecht zur Transportrichtung (T) verlaufende Querschnittsfläche im ersten Bereich (11).

Unter *"Transportrichtung des Abgases"* ist in einem dreidimensionalen Koordinatensystem die x-Achse gemeint. Unter *"senkrecht zur Transportrichtung des Abgases verlaufenden Richtung"* ist in einem dreidimensionalen Koordinatensystem die z-Achse gemeint, welche zum Erdmittelpunkt, dem Ziel der Schwerkraft, verläuft. Unter *"entlang der Transportrichtung des Abgases"* ist die Betrachtungsweise gemeint, unter der das Abgas nach Eintritt in den Kanal (5) zuerst den ersten Bereich (11) passiert, welcher der Eingangsöffnung (10) am nächsten liegt, und den zweiten Bereich (12), welcher der Ausgangsöffnung (20) am nächsten liegt, zuletzt passiert.

Gegenüber den aus dem Stand der Technik bekannten Vorrichtungen ändert sich die Querschnittsfläche im zweiten Bereich (12) gegenüber der aus dem ersten Bereich (11). Unter dem Begriff *"Querschnittsfläche"* ist im Sinne der Anmeldung eine vom Kanal (5) zur Beförderung des Abgases vorgesehene Fläche senkrecht zur Transportrichtung des Abgases zu verstehen. Durch die Änderung der Querschnittsfläche lässt sich in vorteilhafter Weise eine vergleichsweise hohe Abgasgeschwindigkeit, die für ein Abscheiden der Staubpartikel, insbesondere der Harnstoffstaubpartikel, im ersten Bereich (11) günstig ist, im zweiten Bereich (12) gegenüber dem ersten Bereich (11) reduzieren. Ein Resultat der geminderten Abgasgeschwindigkeit ist eine verlängerte Verweildauer im zweiten Bereich (12), welche einen Übergang der genannten chemischen Verbindungen, insbesondere des Ammoniaks, aus der Gasphase in die Flüssigphase und damit das Abscheiden der genannten chemischen Verbindungen, insbesondere des Ammoniaks, begünstigt. Damit lässt sich mit der erfindungsgemäßen Vorrichtung im Besonderen berücksichtigen, dass für die verschiedenen Abscheidungsprozesse im ersten (11) und zweiten Bereich (12) unterschiedliche Abgasgeschwindigkeiten im Hinblick auf Ihren Wirkungsgrad für das Abscheiden von Vorteil sind.

Bevorzugt entspricht eine senkrecht zur Transportrichtung (T) verlaufende Querschnittsfläche des Kanals (5) im ersten Bereich (11) der senkrecht zur Transportrichtung (T) verlaufenden Querschnittsfläche der Eingangsöffnung (10).

Bevorzugt entspricht eine senkrecht zur Transportrichtung (T) verlaufende Querschnittsfläche des Kanals (5) im zweiten Bereich (12) der senkrecht zur Transportrichtung (T) verlaufenden Querschnittsfläche der Ausgangsöffnung (20).

Bevorzugt umfasst der zweite Bereich (12) keine einzige senkrecht zur Transportrichtung (T) verlaufende Querschnittsfläche des Kanals (5), welche kleiner ist als eine senkrecht zur Transportrichtung (T) verlaufende Querschnittsfläche des Kanals (5) im ersten Bereich (11) und/oder welche genauso groß ist wie eine senkrecht zur Transportrichtung (T) verlaufende Querschnittsfläche des Kanals (5) im ersten Bereich (11). Bevorzugt ist die maximale senkrecht zur Transportrichtung (T) verlaufende Querschnittsfläche des Kanals (5) im zweiten Bereich (12) größer als die maximale senkrecht zur Transportrichtung (T) verlaufende Querschnittsfläche des Kanals (5) im ersten Bereich (11).

Bevorzugt umfasst der erste Bereich (11) keine einzige senkrecht zur Transportrichtung (T) verlaufende Querschnittsfläche des Kanals (5), welche größer ist als eine senkrecht zur Transportrichtung (T) verlaufende Querschnittsfläche des Kanals (5) im zweiten Bereich (12) und/oder welche genauso groß ist wie eine senkrecht zur Transportrichtung (T) verlaufende Querschnittsfläche des Kanals (5) im zweiten Bereich (12). Bevorzugt ist die maximale senkrecht zur Transportrichtung (T) verlaufende Querschnittsfläche des Kanals (5) im ersten Bereich (11) kleiner als die maximale senkrecht zur Transportrichtung (T) verlaufende Querschnittsfläche des Kanals (5) im zweiten Bereich (12).

Bevorzugt ist die maximale Querschnittsfläche des Kanals (5) im ersten Bereich (11) mindestens 3 m², oder mindestens 6 m², oder mindestens 9 m², oder mindestens 12 m², oder mindestens 15 m², oder mindestens 18 m², oder mindestens 21 m², oder mindestens 24 m², oder mindestens 27 m², oder mindestens 30 m², oder mindestens 33 m², oder mindestens 36 m², oder mindestens 39 m², oder mindestens 42 m², oder mindestens 45 m². Bevorzugt ist die maximale Querschnittsfläche des Kanals (5) im ersten Bereich (11) kleiner als 45 m², oder 42 m², oder 39 m², oder 36 m², oder 33 m², oder 30 m², oder 27 m², oder 24 m², oder 21 m², oder 18 m², oder 15 m², oder 12 m², oder 9 m², oder 6 m², oder 3 m².

Bevorzugt ist die maximale Querschnittsfläche des Kanals (5) im zweiten Bereich (12) mindestens 3 m², oder mindestens 6 m², oder mindestens 9 m², oder mindestens 12 m², oder mindestens 15 m², oder mindestens 18 m², oder mindestens 21 m², oder mindestens 24 m², oder mindestens 27 m², oder mindestens 30 m², oder mindestens 33 m², oder mindestens 36 m², oder mindestens 39 m², oder mindestens 42 m², oder mindestens 45 m². Bevorzugt ist die maximale Querschnittsfläche des Kanals (5) im zweiten Bereich (12) kleiner als 45 m², oder 42 m², oder 39 m², oder 36 m², oder 33 m², oder 30 m², oder 27 m², oder 24 m², oder 21 m², oder 18 m², oder 15 m², oder 12 m², oder 9 m², oder 6 m², oder 3 m².

Bevorzugt ist eine Querschnittsfläche des Kanals (5) im zweiten Bereich (12) um mindestens 5% oder mindestens 10% oder mindestens 15% oder mindestens 20% oder mindestens 25% oder mindestens 30% oder mindestens 35% oder mindestens 40% oder mindestens 45% oder mindestens 50% oder mindestens 55% oder mindestens 60% oder mindestens 65% oder mindestens 70% größer als die senkrecht zur Transportrichtung (T) verlaufende maximale Querschnittsfläche im ersten Bereich (11). Beträgt die senkrecht zur Transportrichtung (T) verlaufende maximale Querschnittsfläche im ersten Bereich (11) z.B. 10 m², und ist eine Querschnittsfläche des Kanals (5) im zweiten Bereich (12) um mindestens 10% größer, dann beträgt eine senkrecht zur Transportrichtung (T) verlaufende Querschnittsfläche im zweiten Bereich (12) mindestens 11 m².

Bevorzugt ist die maximale Querschnittsfläche des Kanals (5) im ersten Bereich (11) um mindestens 5% oder mindestens 10% oder mindestens 15% oder mindestens 20% oder mindestens 25% oder mindestens 30% oder mindestens 35% oder mindestens 40% oder mindestens 45% oder mindestens 50% oder mindestens 55% oder mindestens 60% oder mindestens 65% oder mindestens 70% kleiner als eine senkrecht zur Transportrichtung (T) verlaufende Querschnittsfläche im zweiten Bereich (12). Beträgt eine senkrecht zur Transportrichtung (T) verlaufende Querschnittsfläche im zweiten Bereich (12) z.B. 10 m², und ist die maximale Querschnittsfläche des Kanals (5) im ersten Bereich (11) um mindestens 10% kleiner, dann beträgt die senkrecht zur Transportrichtung (T) verlaufende maximale Querschnittsfläche im ersten Bereich (11) höchstens 9 m².

Bevorzugt ist die Querschnittsfläche des Kanals (5) im zweiten Bereich (12) um mindestens 5% und höchstens 10%; oder mindestens 10% und höchstens 20%; oder mindestens 15% und höchstens 30%; oder mindestens 20% und höchstens 40%; oder mindestens 25% und höchstens 50%; oder mindestens 30% und höchstens 60%; oder mindestens 35% und höchstens 70%; oder mindestens 40% und höchstens 80%; oder mindestens 45% und höchstens 90% größer als die senkrecht zur Transportrichtung (T) verlaufende maximale Querschnittsfläche im ersten Bereich (11). Beträgt die senkrecht zur Transportrichtung (T) verlaufende maximale Querschnittsfläche im ersten Bereich (11) z.B. 10 m², und ist eine Querschnittsfläche des Kanals (5) im zweiten Bereich (12) um mindestens 10% und höchstens 20% größer, dann beträgt eine senkrecht zur Transportrichtung (T) verlaufende Querschnittsfläche im zweiten Bereich (12) mindestens 11 m² und höchstens 12 m².

Bevorzugt ist die maximale Querschnittsfläche des Kanals im ersten Bereich (11) um mindestens 5% und höchstens 10%; oder mindestens 10% und höchstens 20%; oder mindestens 15% und höchstens 30%; oder mindestens 20% und höchstens 40%; oder mindestens 25% und höchstens 50%; oder mindestens 30% und höchstens 60%; oder mindestens 35% und höchstens 70%; oder mindestens 40% und höchstens 80%; oder mindestens 45% und höchstens 90% kleiner als eine senkrecht zur Transportrichtung (T) verlaufende Querschnittsfläche im zweiten Bereich (12). Beträgt eine senkrecht zur Transportrichtung (T) verlaufende Querschnittsfläche im zweiten Bereich (12) z.B. 10 m², und ist die maximale Querschnittsfläche des Kanals im ersten Bereich (11) um mindestens 10% und höchstens 20% kleiner, dann beträgt die senkrecht zur Transportrichtung (T) verlaufende maximale Querschnittsfläche im ersten Bereich (11) mindestens 8 m² und höchstens 9 m².

Bevorzugt erfolgt eine Änderung der Querschnittsfläche zwischen dem ersten Bereich (11) und dem zweiten Bereich (12) in einer entlang der Transportrichtung (T) des Abgases verlaufenden Richtung über einen stufenförmigen oder einen schrägen Verlauf in einer Kanalwand oder mehreren Kanalwänden des Kanals (5). Bevorzugt ist die Änderung der Querschnittsfläche an die jeweilige bauraumgegebene Situation einer Harnstoffanlage angepasst.

Bevorzugt beträgt die Länge des Kanals (5) mindestens 2 m, oder mindestens 4 m, oder mindestens 6 m, oder mindestens 8 m, oder mindestens 10 m, oder mindestens 12 m, oder mindestens 14 m, oder mindestens 16 m oder mindestens 18 m oder mindestens 20 m, oder mindestens 22 m, oder mindestens 24 m oder mindestens 26 m oder mindestens 28 m oder mindestens 30 m oder mindestens 32 m. Bevorzugt beträgt die Länge des Kanals (5) 2 m bis 32 m, oder 4 m bis 30 m, oder 6 m bis 28 m, oder 8 m bis 26 m, oder 10 m bis 24 m, oder 12 m bis 22 m, oder 14 m bis 20 m, oder 16 m bis 18 m. Bevorzugt beträgt die Länge des Kanals (5) weniger als 32 m, oder weniger als 28 m, oder weniger als 24 m, oder weniger als 20 m, oder weniger als 16 m, oder weniger als 12 m, oder weniger als 8 m, oder weniger als 4 m.

Bevorzugt ist die Vorrichtung derart ausgestaltet, dass der Kanal (5) im verbauten Zustand der Vorrichtung, insbesondere integriert in einer Harnstoffanlage, im Wesentlichen horizontal oder im Wesentlichen vertikal verläuft. Unter *"horizontal* ist in einem dreidimensionalen Koordinatensystem (eine Parallele zur) x-Achse gemeint. Unter *"vertikal"* ist in einem dreidimensionalen Koordinatensystem die z-Achse gemeint, welche zum Erdmittelpunkt, dem Ziel der Schwerkraft, verläuft.

Bevorzugt beträgt die Länge des ersten Bereichs (11) mindestens 2 m, oder mindestens 4 m, oder mindestens 6 m, oder mindestens 8 m, oder mindestens 10 m. Bevorzugt beträgt die Länge des zweiten Bereichs (12) mindestens 2 m, oder mindestens 4 m, oder mindestens 6 m, oder mindestens 8 m, oder mindestens 10 m. Bevorzugt ist der zweite Bereich (12) länger als der erste Bereich (11). Bevorzugt ist der erste Bereich (11) länger als der zweite Bereich (12). Bevorzugt sind der erste Bereich (11) und der zweite Bereich (12) gleich lang. Bevorzugt wird das Abgas mit einer Geschwindigkeit von mehr als 1 m/s, vorzugsweise mehr als 5 m/s und besonders bevorzugt mehr als 10 m/s durch den ersten Bereich (11) transportiert. Bevorzugt wird das Abgas mit einer Geschwindigkeit von weniger als 20 m/s, vorzugsweise weniger als 10 m/s und besonders bevorzugt weniger als 5 m/s durch den zweiten Bereich (12) transportiert. Bevorzugt wird das Abgas mit einer Geschwindigkeit von mehr als 10 m/s durch den ersten Bereich (11) und von weniger als 10 m/s durch den zweiten Bereich (12) transportiert, oder mit einer Geschwindigkeit von mehr als 5 m/s durch den ersten Bereich (11) und von weniger als 5 m/s durch den zweiten Bereich (12) transportiert, oder mit einer Geschwindigkeit von mehr als 1 m/s durch den ersten Bereich (11) und von weniger als 1 m/s durch den zweiten Bereich (12) transportiert. Bevorzugt ist die Abgasgeschwindigkeit im zweiten Bereich (12) zu jedem Zeitpunkt der Abgaswäsche geringer als die Abgasgeschwindigkeit im ersten Bereich (11). Bevorzugt ist die Abgasgeschwindigkeit im ersten Bereich (11) zu jedem Zeitpunkt der Abgaswäsche größer als die Abgasgeschwindigkeit im zweiten Bereich (12).

Bevorzugt weist das durch Eingangsöffnung (10) eintretende Abgas eine Temperatur von maximal 140 °C, bevorzugt maximal 120 °C, bevorzugt maximal 100 °C, bevorzugt maximal 80 °C, bevorzugt maximal 60 °C, oder bevorzugt maximal 50 °C auf.

Bevorzugt weisen die im ersten Bereich (11) zu entfernenden Staubpartikel einen Durchmesser von größer als 1 µm auf.

Erfindungsgemäß ist die Vorrichtung (1) in einer Harnstoffanlage integriert, und das Abgas der Harnstoffanlage wird durch die Eingangsöffnung (10) in die Vorrichtung (1) eingeleitet und das gereinigte Abgas verlässt über die Ausgangsöffnung (20) die Vorrichtung (1) wieder. In diesem Fall wird im ersten Bereich (11) Harnstoffstaub abgeschieden. Dieser unterscheidet sich von anderen im industriellen Betrieb anfallenden Stäuben insbesondere durch seine Wasserlöslichkeit. Harnstoffstaub ist sehr gut löslich in Wasser und auch in Harnstofflösung. Ferner ist es bei Harnstoffstaub möglich, dass sich im Laufe der Besprühung mit Harnstofflösung bzw. Wasser größere Partikel aufgrund des *"Abwaschens"* äußerer Schichten zu kleineren Harnstoffstaubpartikeln umwandeln. Dies ist bei nicht-wasserlöslichen Staubpartikeln nicht möglich. Hinsichtlich der Geometrie der Harnstoffstaubpartikel sind diese aufgrund ihrer Genese im Wesentlichen rund. Staubpartikel aus anderen Verfahren (z.B. Rauchgasentschwefelungen; CO₂-Entfernung aus dem Rauchgas fossil-gefeuerter Kraftwerke; oder saure Aufbereitung fluorid-haltiger Erze) können anders geformt sein (eckig, verzwillingt, agglomeriert, etc.). Im zweiten Bereich (12) wird im Falle der Integration in einer Harnstoffanlage Ammoniak abgeschieden.

[Die folgenden bevorzugten Ausführungsformen sind dahingehend auszulegen, dass sie für die Bereiche (11, 12), für die jeweiligen Oberflächenvergrößerungsstrukturen (31, 32), für die jeweils enthaltenen Sprühvorrichtungen (2) und für die jeweiligen verwendeten Lösungsmittel (21, 22) unabhängig voneinander gelten, sofern nicht explizit auf etwas Anderes hingewiesen wird. Z.B. ist unter *"bevorzugt umfasst die Sprühvorrichtung (2) zum Versprühen eines Lösungsmittels (21, 22) eine oder mehrere Sprühdüsen"* eine Sprühvorrichtung (2) zu verstehen, welche sich ausschließlich im ersten Bereich (11) zum Versprühen eines ersten Lösungsmittels (21) befindet; oder eine Sprühvorrichtung (2) zu verstehen, welche sich ausschließlich im zweiten Bereich (12) zum Versprühen eines zweiten Lösungsmittels (22) befindet; oder Sprühvorrichtungen (2) zu verstehen, welche sich im ersten Bereich (11) zum Versprühen eines ersten Lösungsmittels (21) und im zweiten Bereich (12) zum Versprühen eines zweiten Lösungsmittels (22) befinden.

Erfindungsgemäß umfasst der erste Bereich (11) eine Sprühvorrichtung (2) zum Versprühen eines ersten Lösungsmittels (21) und der zweite Bereich (12) eine Sprühvorrichtung (2) zum Versprühen eines zweiten Lösungsmittels (22), d.h. der erste Bereich (11) umfasst eine in dem Kanal (5) angeordnete erste Oberflächenvergrößerungsstruktur (31) zur Entfernung von Staubpartikeln, insbesondere von Harnstoffstaubpartikeln, und der zweite Bereich (12) umfasst eine in dem Kanal (5) angeordnete zweite Oberflächenvergrößerungsstruktur (32) zur Entfernung der genannten chemischen Verbindungen, insbesondere von Ammoniak. Dadurch dass die Oberflächenvergrößerungsstrukturen (31, 32) mit dem ersten Lösungsmittel (21) und/oder dem zweiten Lösungsmittel (22) besprüht werden, werden in vorteilhafter Weise an den Oberflächenvergrößerungsstrukturen (31, 32) abgelagerte Staubpartikel, insbesondere Harnstoffstaubpartikel, abgewaschen bzw. gehen die genannten chemischen Verbindungen, insbesondere Ammoniak, aus der Gasphase an dem durch den sich auf den Oberflächenvergrößerungsstrukturen (31, 32) bildenden Film in die jeweilige Waschlösung über, wodurch die abgewaschenen Bereiche der Oberflächenvergrößerungsstrukturen (31, 32) für ein erneutes Ablagern der Staubpartikel, insbesondere Harnstoffstaubpartikel, und/oder der genannten chemischen Verbindungen, insbesondere Ammoniak, dem Abgas bereitgestellt werden können. Es ist anzumerken, dass Ammoniak nicht mit gleichen physikalischen Eigenschaften wie Harnstoffstaub abgeschieden wird.

Bevorzugt umfasst der erste Bereich (11) zur Entfernung von Staubpartikeln, insbesondere von Harnstoffstaubpartikeln, mehrere Stufen und/oder der zweite Bereich (12) zur Entfernung der genannten chemischen Verbindungen, insbesondere von Ammoniak, mehrere Stufen. In diesem Fall umfasst der erste Bereich (11) und/oder der zweite Bereich (12) mehrere Oberflächenvergrößerungsstrukturen (31, 32), wobei die ersten Oberflächenvergrößerungsstrukturen (31) im ersten Bereich (11) jeweils mit einem ersten Lösungsmittel (21) besprüht werden und/oder die zweiten Oberflächenvergrößerungsstrukturen (32) im zweiten Bereich (12) jeweils mit einem zweiten Lösungsmittel (22) besprüht werden. Mittels der einzelnen Stufen kann der Staubpartikelgehalt, insbesondere der von Harnstoffstaubpartikeln, und/oder der Gehalt der genannten chemischen Verbindungen, insbesondere der Ammoniakgehalt, weiter reduziert werden. Dabei sind die einzelnen Stufen entlang der Transportrichtung (T) des Abgases hintereinander angeordnet.

Bevorzugt umfasst die Sprühvorrichtung (2) zum Versprühen eines Lösungsmittels (21, 22) eine oder mehrere Sprühdüsen. Bevorzugt umfasst die Sprühvorrichtung (2) eine oder zwei oder drei oder vier oder fünf oder sechs oder sieben oder acht oder neun oder zehn Sprühdüsen. Bevorzugt umfasst die Sprühvorrichtung (2) mindestens eine oder mindestens zwei oder mindestens drei oder mindestens vier oder mindestens fünf oder mindestens sechs oder mindestens sieben oder mindestens acht oder mindestens neun oder mindestens zehn Sprühdüsen. Bevorzugt umfasst die Sprühvorrichtung (2) mindestens eine und maximal zehn Sprühdüsen, oder mindestens zwei und maximal neun Sprühdüsen, oder mindestens drei und maximal acht Sprühdüsen, oder mindestens vier und maximal sieben Sprühdüsen.

Bevorzugt umfasst die Sprühvorrichtung (2) zum Versprühen eines Lösungsmittels (21, 22) ein Verteilerrohr. Ein Verteilerrohr ist eine Vorrichtung, in welche mehrere Sprühdüsen integriert sind und über eine gemeinsame Leitung mit Lösungsmittel (21, 22) versorgt werden. Bevorzugt umfasst ein Verteilerrohr zwei oder drei oder vier oder fünf oder sechs oder sieben oder acht oder neun oder zehn Sprühdüsen. Bevorzugt umfasst ein Verteilerrohr mindestens zwei oder mindestens drei oder mindestens vier oder mindestens fünf oder mindestens sechs oder mindestens sieben oder mindestens acht oder mindestens neun oder mindestens zehn Sprühdüsen. Bevorzugt umfasst ein Verteilerrohr mindestens zwei und maximal zehn Sprühdüsen, oder mindestens drei und maximal neun Sprühdüsen, oder mindestens vier und maximal acht Sprühdüsen, oder mindestens fünf und maximal sieben Sprühdüsen.

Bevorzugt umfasst die Sprühvorrichtung (2) zum Versprühen eines Lösungsmittels (21, 22) mehrere Verteilerrohre. Bevorzugt umfasst die Sprühvorrichtung (2) zwei oder drei oder vier oder fünf oder sechs oder sieben oder acht oder neun oder zehn Verteilerrohre. Bevorzugt umfasst jedes einzelne Verteilerrohr zwei oder drei oder vier oder fünf oder sechs oder sieben oder acht oder neun oder zehn Sprühdüsen. Bevorzugt umfasst jedes einzelne Verteilerrohr mindestens zwei oder mindestens drei oder mindestens vier oder mindestens fünf oder mindestens sechs oder mindestens sieben oder mindestens acht oder mindestens neun oder mindestens zehn Sprühdüsen. Bevorzugt umfasst jedes einzelne Verteilerrohr mindestens zwei und maximal zehn Sprühdüsen, oder mindestens drei und maximal neun Sprühdüsen, oder mindestens vier und maximal acht Sprühdüsen, oder mindestens fünf und maximal sieben Sprühdüsen.

Bevorzugt umfasst die Sprühvorrichtung (2) zum Versprühen eines Lösungsmittels (21, 22) zwei bis zehn Verteilerrohre, welche mindestens zwanzig Sprühdüsen umfassen. Bevorzugt umfasst die Sprühvorrichtung (2) zwei bis neun Verteilerrohre, welche mindestens achtzehn Sprühdüsen umfassen. Bevorzugt umfasst die Sprühvorrichtung (2) zwei bis acht Verteilerrohre, welche mindestens sechszehn Sprühdüsen umfassen. Bevorzugt umfasst die Sprühvorrichtung (2) zwei bis sieben Verteilerrohre, welche mindestens vierzehn Sprühdüsen umfassen. Bevorzugt umfasst die Sprühvorrichtung (2) zwei bis sechs Verteilerrohre, welche mindestens zwölf Sprühdüsen umfassen. Bevorzugt umfasst die Sprühvorrichtung (2) zwei bis fünf Verteilerrohre, welche mindestens zehn Sprühdüsen umfassen. Bevorzugt umfasst die Sprühvorrichtung (2) zwei bis vier Verteilerrohre, welche mindestens acht Sprühdüsen umfassen. Bevorzugt umfasst die Sprühvorrichtung (2) zwei bis drei Verteilerrohre, welche mindestens sechs Sprühdüsen umfassen.

Bevorzugt umfasst die Sprühvorrichtung (2) zum Versprühen eines Lösungsmittels (21, 22) ein oder mehrere Verteilerrohre, welche mehrere Sprühdüsen umfassen, und eine oder mehrere Sprühdüsen, welche nicht von Verteilerrohren umfasst werden. Bevorzugt umfasst die Sprühvorrichtung (2) mindestens ein Verteilerrohr, welches mindestens zwei Sprühdüsen umfasst, und mindestens eine Sprühdüse, welche nicht von Verteilerrohren umfasst wird. Bevorzugt umfasst die Sprühvorrichtung (2) mindestens zwei Verteilerrohre, welche mindestens vier Sprühdüsen umfassen, und mindestens zwei Sprühdüsen, welche nicht von Verteilerrohren umfasst werden. Bevorzugt umfasst die Sprühvorrichtung (2) mindestens drei Verteilerrohre, welche mindestens sechs Sprühdüsen umfassen, und mindestens drei Sprühdüsen, welche nicht von Verteilerrohren umfasst werden. Bevorzugt umfasst die Sprühvorrichtung (2) mindestens vier Verteilerrohre, welche mindestens acht Sprühdüsen umfassen, und mindestens vier Sprühdüsen, welche nicht von Verteilerrohren umfasst werden. Bevorzugt umfasst die Sprühvorrichtung (2) mindestens fünf Verteilerrohre, welche mindestens zehn Sprühdüsen umfassen, und mindestens fünf Sprühdüsen, welche nicht von Verteilerrohren umfasst werden. Bevorzugt umfasst die Sprühvorrichtung (2) mindestens sechs Verteilerrohre, welche mindestens zwölf Sprühdüsen umfassen, und mindestens sechs Sprühdüsen, welche nicht von Verteilerrohren umfasst werden.

Bevorzugt sind die Sprühdüsen vom selben Typ. Bevorzugt sind sämtliche Sprühdüsen entweder Hohlkegeldüsen oder Vollkegeldüsen oder Flachstrahldüsen oder Vollstrahldüsen oder Spiraldüsen oder Zerstäuberdüsen.

Bevorzugt ist mindestens eine Sprühdüse eine Hohlkegeldüse. Bevorzugt ist mindestens eine Sprühdüse eine Vollkegeldüse. Bevorzugt ist mindestens eine Sprühdüse eine Flachstrahldüse. Bevorzugt ist mindestens eine Sprühdüse eine Vollstrahldüse. Bevorzugt ist mindestens eine Sprühdüse eine Spiraldüse. Bevorzugt ist mindestens eine Sprühdüse eine Zerstäuberdüse.
Bevorzugt sind die Sprühdüsen unterschiedlichen Typs. Bevorzugt ist maximal eine Sprühdüse eine Vollkegeldüse. Bevorzugt ist maximal eine Sprühdüse eine Flachstrahldüse. Bevorzugt ist maximal eine Sprühdüse eine Vollstrahldüse. Bevorzugt ist maximal eine Sprühdüse eine Spiraldüse. Bevorzugt ist maximal eine Sprühdüse eine Zerstäuberdüse.

Bevorzugt wird gasförmiges Ammoniak in die wässrige Flüssigphase integriert und aus der Vorrichtung (1) entfernt. In der Flüssigphase reagiert das Ammoniak instantan mit einer sauren Waschlösung und bildet Ammonium-Ionen. Durch diese Ammonium-Ionen-Bildung ist der Ammoniak-Partialdruck über der Flüssigphase in etwa null, so dass es möglich ist das Ammoniak aus der Gasphase zu entfernen.

Besonders bevorzugt wird gemäß der vorliegenden Erfindung in dem zweiten Bereich Ammoniak aus dem Gasstrom entfernt, das heißt es erfolgt eine saure Wäsche.

Bevorzugt wird
- gasförmiger Ammoniak, und/oder
- gasförmiges NOₓ, und/oder
- gasförmiges NO₂, und/oder
- gasförmiges SO₂, insbesondere bei Rauchgasentschwefelungen, und/oder
- gasförmiges CO₂, insbesondere bei der CO₂-Entfernung aus dem Rauchgas fossil-gefeuerter Kraftwerke, und/oder
- HF, insbesondere bei der sauren Aufbereitung fluorid-haltiger Erze, beispielsweise Phosphaterze,
in die wässrige Flüssigphase integriert und aus der Vorrichtung (1) entfernt.

Bevorzugt werden gasförmige reaktive Komponenten, bevorzugt organische reaktive Komponenten, in die wässrige Flüssigphase integriert und aus der Vorrichtung (1) entfernt.

Das zweite Lösungsmittel (22) umfasst mindestens eine Komponente, welche die gasförmige chemische Verbindung in die wässrige Flüssigphase überführt.

Bevorzugt umfasst das zweite Lösungsmittel (22) eine saure Waschlösung. Bevorzugt umfasst die saure Waschlösung Schwefelsäure, Salpetersäure, Salzsäure, Phosphorsäure, oder Mischungen aus Schwefelsäure, Salpetersäure, Salzsäure und/oder Phosphorsäure, oder Mischungen aus Schwefelsäure, Salpetersäure, Salzsäure, Phosphorsäure und/oder Harnstofflösung. Der Säuregehalt einer der vorgenannten Säuren oder der Mischungen von mindestens zwei der vorgenannten Säuren liegt bevorzugt im Bereich von 0,01 - 50,00 Gew.-%, oder im Bereich von 0,01 - 10,00 Gew.-% oder im Bereich von 0,1 - 5,00 Gew.-%, bezogen auf das Gesamtgewicht des zweiten Lösungsmittels (22). Für die Ammoniakabscheidung geeignete Säuren haben bevorzugt einen pk(S)-Wert kleiner als 7, oder kleiner als 5 oder kleiner als 2,5.

Bevorzugt umfasst das zweite Lösungsmittel (22) eine Base. Bevorzugt umfasst das zweite Lösungsmittel (22) eine starke und/oder eine schwache Base. Bevorzugt umfasst das zweite Lösungsmittel (22) eine starke anorganische und/oder eine schwache anorganische und/oder eine starke organische und/oder eine schwache organische Base. Bevorzugt umfasst das zweite Lösungsmittel (22) Natriumhydroxid (NaOH), Kaliumhydroxid (KOH), Ammoniaklösungen und/oder Calciumhydroxid (Ca(OH)₂).

Bevorzugt umfasst das zweite Lösungsmittel (22) mono-, bi- und/oder trifunktionale Amine, bevorzugt MDEA (Methyldiethanolamin).

Bevorzugt umfasst das erste Lösungsmittel (21) Wasser und/oder eine Harnstofflösung. Bevorzugt umfasst das erste Lösungsmittel (21) ausschließlich Wasser oder ausschließlich eine Harnstofflösung.

Bevorzugt unterscheiden sich das erste Lösungsmittel (21) und das zweite Lösungsmittel (22) dahingehend, dass das erste Lösungsmittel (21) mindestens eine Komponente umfasst, welche das zweite Lösungsmittel (22) nicht umfasst. Bevorzugt unterscheiden sich das erste Lösungsmittel (21) und das zweite Lösungsmittel (22) dahingehend, dass das zweite Lösungsmittel (22) mindestens eine Komponente umfasst, welche das erste Lösungsmittel (21) nicht umfasst.

Bevorzugt wird das erste (21) und/oder das zweite Lösungsmittel (22) von oben oder von schräg unten auf die Oberflächenvergrößerungsstrukturen (31, 32) gegeben.

Bevorzugt wird eine Waschlösung, welche von einer der Oberflächenvergrößerungsstrukturen (31, 32) abläuft, für ein erneutes Besprühen der Oberflächenvergrößerungsstrukturen (31, 32) verwendet, d.h. als erstes (21) und/oder zweites Lösungsmittel (22) in vorteilhafter Weise wiederverwertet. Hierzu wird die abgelaufene Waschlösung gesammelt und der Sprühvorrichtung (2) wieder zugeführt. Möglich ist auch, dass nur ein Teil der gesammelten Waschlösung, dieser jedoch mit Frischwasser und/oder Frischsäure und/oder Frischbase aufgestockt, verwendet und zurückgeführt wird.

Bevorzugt umfasst die Oberflächenvergrößerungsstruktur (31, 32) Einbauten, welche zu einer entsprechenden Vergrößerung der Oberfläche führen, dabei jedoch dem Abgas aufgrund des erzeugten Druckverlustes nur geringen Widerstand entgegensetzen. Bevorzugt umfasst die Oberflächenvergrößerungsstruktur (31, 32) regellose Schüttungen und/oder strukturierte Packungen. Bevorzugt umfasst die Oberflächenvergrößerungsstruktur (31, 32) regellose Schüttungen ausgewählt aus der Gruppe bestehend aus Raschig-Ringen, Sattelkörpern, Spiralen und Kombinationen davon. Bevorzugt umfasst die Oberflächenvergrößerungsstruktur (31, 32) strukturierte Packungen ausgewählt aus der Gruppe bestehend aus Gewebepackungen, Blechpackungen, Streckmetallpackungen, Kunststoffpackungen, Gridpackungen und Kombinationen davon. Bevorzugt umfasst die Oberflächenvergrößerungsstruktur (31, 32) regellose Schüttungen ausgewählt aus der Gruppe bestehend aus Raschig-Ringen, Sattelkörpern, Spiralen und Kombinationen davon, und strukturierte Packungen ausgewählt aus der Gruppe bestehend aus Gewebepackungen, Blechpackungen, Streckmetallpackungen, Kunststoffpackungen, Gridpackungen und Kombinationen davon. Bevorzugt umfasst die Oberflächenvergrößerungsstruktur (31, 32)
- regellose Strukturelemente ausgewählt aus der Gruppe bestehend aus Raschig-Ringen, Pall-Ringen, Sattel-Körpern, Feder-typischen Körpern und Kombinationen davon; und/oder
- strukturierte Strukturelemente ausgewählt aus der Gruppe bestehend aus Blechen, gewellten Blechen, geformten Blechen und Kombinationen davon; und/oder
- Materialien ausgewählt aus der Gruppe bestehend aus Metall, Glas, Kunststoff, Carbonfasern und Kombinationen davon.

Ein zweiter Aspekt der vorliegenden Erfindung ist ein Verfahren zur Abgaswäsche in einer Harnstoffanlage, wobei ein Abgas durch einen Kanal (5) mit einem ersten Bereich (11) zur Entfernung von Staubpartikeln, insbesondere von Harnstoffstaubpartikeln, und einen zweiten Bereich (12) zur Entfernung der genannten chemischen Verbindungen, insbesondere von Ammoniak, geführt wird, wobei eine Abgasgeschwindigkeit, mit der das Abgas im Kanal (5) geführt wird, im ersten Bereich (11) größer ist als im zweiten Bereich (12).

Gegenüber den aus dem Stand der Technik bekannten Verfahren hat das erfindungsgemäße Verfahren den Vorteil, dass sich durch die reduzierte Abgasgeschwindigkeit im zweiten Bereich (12) ein erhöhter Wirkungsgrad beim Abscheiden der genannten chemischen Verbindungen, insbesondere von Ammoniak, erzielen lässt. Die reduzierte Abgasgeschwindigkeit im zweiten Bereich (12) wird durch eine Vergrößerung der Querschnittsfläche des Kanals (5) im zweiten Bereich (12) gegenüber dem ersten Bereich (11) realisiert.

Ein dritter Aspekt der vorliegenden Erfindung ist die Verwendung der erfindungsgemäßen Vorrichtung zur Abgaswäsche.

Weitere Einzelheiten, Merkmale und Vorteile der Erfindung ergeben sich aus den Zeichnungen, sowie aus der nachfolgenden Beschreibung von bevorzugten Ausführungsformen anhand der Zeichnungen. Die Zeichnungen illustrieren dabei lediglich beispielhafte Ausführungsformen der Erfindung, welche den Erfindungsgedanken nicht einschränken. Dabei zeigen:
Figur 1 eine Vorrichtung zur Abgaswäsche gemäß einer ersten beispielhaften Ausführungsform der vorliegenden Erfindung;
Figur 2A eine Vorrichtung zur Abgaswäsche gemäß einer zweiten beispielhaften Ausführungsform der vorliegenden Erfindung in der Draufsicht;
Figur 2B die Vorrichtung gemäß Figur 2A in der Seitenansicht;
Figur 3A eine Vorrichtung zur Abgaswäsche gemäß einer dritten beispielhaften Ausführungsform der vorliegenden Erfindung in der Draufsicht;
Figur 3B die Vorrichtung gemäß Figur 3A in der Seitenansicht;
Figur 4A eine Vorrichtung zur Abgaswäsche gemäß einer vierten beispielhaften Ausführungsform der vorliegenden Erfindung in der Draufsicht;
Figur 4B die Vorrichtung gemäß Figur 4A in der Seitenansicht;
Figur 5A eine Vorrichtung zur Abgaswäsche gemäß einer fünften beispielhaften Ausführungsform der vorliegenden Erfindung in der Draufsicht;
Figur 5B die Vorrichtung gemäß Figur 5A in der Seitenansicht;
Figur 6A eine Vorrichtung zur Abgaswäsche gemäß einer sechsten beispielhaften Ausführungsform der vorliegenden Erfindung in der Draufsicht;
Figur 6B die Vorrichtung gemäß Figur 6A in der Seitenansicht;
Figur 7A eine Vorrichtung zur Abgaswäsche gemäß einer siebten beispielhaften Ausführungsform der vorliegenden Erfindung in der Draufsicht;
Figur 7B die Vorrichtung gemäß Figur 7A in der Seitenansicht. In Figur 1 ist eine Vorrichtung (1) zur Abgaswäsche gemäß einer ersten beispielhaften Ausführungsform der vorliegenden Erfindung dargestellt. Insbesondere handelt es sich um eine Vorrichtung (1), die in einer Harnstoffanlage eingebaut ist und mit welcher der Zweck verfolgt wird, aus dem Abgas Harnstoffstaubpartikel und Ammoniak soweit zu entfernen, dass ihr verbleibender Anteil im Abgas unterhalb eines kritischen bzw. behördlich vorgeschriebenen Schwellwerts liegt.

Hierzu wird das Abgas über eine Eingangsöffnung (10) in einen Kanal (5) der Vorrichtung (1) eingeführt. Auf dem Weg zur Ausgangsöffnung (20) der Vorrichtung (1) passiert das Abgas entlang einer Transportrichtung (T) im Kanal (5) einen ersten Bereich (11) und einen zweiten Bereich (12). Hierbei ist es vorgesehen, dass im ersten Bereich (11) das Abgas von Harnstoffstaubpartikeln befreit wird, während im zweiten Bereich (12) das Ammoniak aus dem Abgas entfernt wird. Der zweite Bereich (12) ist entlang der Transportrichtung (T) hinter dem ersten Bereich (11) angeordnet.

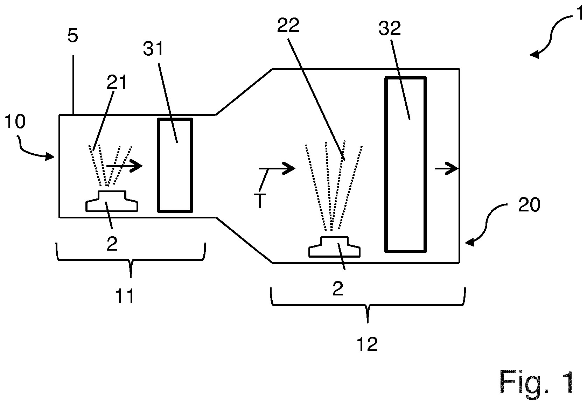

Weiterhin ist für das in der Figur 1 dargestellte Ausführungsbeispiel vorgesehen, dass der Kanal (5) im verbauten Zustand im Wesentlichen horizontal verläuft. Vorrichtungen dieser Art werden auch als Horizontalwäscher bezeichnet.

Zur Entfernung der Harnstoffstaubpartikel wird hierbei eine erste Oberflächenvergrößerungsstruktur (31), durch welches das Abgas geleitet wird, im ersten Bereich (11) mit Hilfe einer Sprühvorrichtung (2) mit einem ersten Lösungsmittel (21) besprüht. Vorzugsweise handelt es sich bei dem ersten Lösungsmittel (21) um Wasser oder eine Harnstofflösung. Denkbar ist, dass das Lösungsmittel (21) von oben auf die erste Oberflächenvergrößerungsstruktur (31) des ersten Bereichs (11) aufgegeben wird und dann schwerkraftgetrieben an dieser herunterläuft (Prinzip des Gradierwerkes). Die Besprühung kann prinzipiell auch von hinten, also im Gegenstrom zur Transportrichtung (T) verlaufen. Die Besprühung kann prinzipiell auch von vorne, also im Gleichstrom zur Transportrichtung (T) verlaufen. Grundsätzlich kann die Besprühung auch von der Seite her oder von unten her quer zum Abgasstrom erfolgen.

Die, insbesondere fein strukturierte, erste Oberflächenvergrößerungsstruktur (31) bewirkt, dass durch ihren innewohnenden, gegebenen Impuls die Harnstoffstaubpartikel auf einer Oberfläche der ersten Oberflächenvergrößerungsstruktur (31) abgeschieden werden.

Vorzugsweise ist die erste Oberflächenvergrößerungsstruktur (31) im ersten Bereich (11) derart ausgestaltet, dass sie einerseits eine möglichst große Oberfläche aufweist und andererseits dem Abgas aufgrund des erzeugten Druckverlusts nur einen vergleichsweise geringen Widerstand entgegensetzt. Vorstellbar sind beispielsweise regellose Schüttungen oder strukturierte Packungen.

Bevorzugt ist vorgesehen, dass mittels weiterer Sprühvorrichtungen (2) (nicht dargestellt) die erste Oberflächenvergrößerungsstruktur (31) mit Wasser oder einer Harnstofflösung besprüht wird. Hierdurch wird in vorteilhafter Weise dafür gesorgt, dass im ersten Bereich (11) die an der Oberfläche abgeschiedenen Harnstoffstaubpartikel von der erste Oberflächenvergrößerungsstruktur (31) abgewaschen werden und somit die Oberfläche für eine erneute Abscheidung weiterer Harnstoffstaubpartikel zur Verfügung gestellt werden kann. Dieses *"Freiräumen"* der Oberfläche gilt insbesondere für den ersten Bereich (11).

Da die Effektivität der Staubabscheidung umso größer ist je höher der Impuls und damit die Geschwindigkeit der Harnstoffstaubpartikel ist, sind im ersten Bereich (11) vergleichsweise hohe Geschwindigkeiten von Vorteil.

Zur Entfernung von Ammoniak wird die zweite Oberflächenvergrößerungsstruktur (32) im zweiten Bereich (12) mit Hilfe einer Sprühvorrichtung (2), vorzugsweise mit mehreren, insbesondere gleichartigen Sprühvorrichtungen (2), mit einem zweiten Lösungsmittel (22) besprüht. Im zweiten Bereich (12) wird dem Gas immer wieder frische, d. h. an Säure unverbrauchte Waschlösung bereitgestellt. Denkbar ist, dass das Lösungsmittel (22) von oben auf die zweite Oberflächenvergrößerungsstruktur (32) des zweiten Bereichs (12) aufgegeben wird und dann schwerkraftgetrieben an dieser herunterläuft (Prinzip des Gradierwerkes). Die Besprühung kann prinzipiell auch von hinten, also im Gegenstrom zur Transportrichtung (T) verlaufen. Die Besprühung kann prinzipiell auch von vorne, also im Gleichstrom zur Transportrichtung (T) verlaufen. Vorzugsweise ist die zweite Oberflächenvergrößerungsstruktur (32) im zweiten Bereich (22) derart ausgestaltet, dass sie einerseits eine möglichst große Oberfläche aufweist und andererseits dem Abgas aufgrund des erzeugten Druckverlusts nur einen vergleichsweise geringen Widerstand entgegensetzt. Vorstellbar sind beispielsweise Schüttungen oder strukturierte Packungen.

Vorzugsweise handelt es sich bei dem zweiten Lösungsmittel (22) um eine saure Waschlösung, wodurch es zu einem erhöhten Stofftransport des Ammoniaks aus der Gasphase in die Flüssigphase kommt. Beispielsweise umfasst die saure Waschlösung eine Säure wie Schwefelsäure, Salpetersäure, Salzsäure oder Phosphorsäure. In der Flüssigphase reagiert das Ammoniak instantan mit der sauren Waschlösung und bildet Ammonium-Ionen. Durch diese Ammonium-Ionen-Bildung ist der Ammoniak-Partialdruck über der Flüssigphase in etwa null, so dass es möglich ist das Ammoniak im Wesentlichen aus der Gasphase zu entfernen. Vorzugsweise wird das Ammoniak im zweiten Bereich (12) mehrstufig entfernt, wobei jede Stufe mindestens eine zweite Oberflächenvergrößerungsstruktur (32) und mindestens eine Sprühvorrichtung (2) umfasst. Hierbei ist es insbesondere vorgesehen, dass eine von der zweiten Oberflächenvergrößerungsstruktur (32) abgelaufene Waschlösung vereinigt bzw. gesammelt wird, und nach einer Erhöhung der Säurekonzentration erneut auf die zweite Oberflächenvergrößerungsstruktur (32) gesprüht wird. Dadurch lässt sich die abgelaufene Waschlösung in vorteilhafter Weise als zweites Lösungsmittel (22) wiederverwerten. Beispielsweise ist die Sprühvorrichtung (2) als eine einzelne Düse, als eine Mehrstockdüse, als eine Kegelmusterdüse, als eine Düse zur Bereitstellung eines quadratischen Musters, als eine Düse zur Bereitstellung eines Strahls oder als eine eine zufällige Lösungsmittelverteilung hervorrufende Düse ausgestaltet.

Um das Entfernen des Ammoniaks aus dem Abgas zu verbessern, ist es insbesondere vorgesehen, dass eine Abgasgeschwindigkeit, mit der das Abgas durch den Kanal (5) geführt wird, im zweiten Bereich (12) gegenüber der Abgasgeschwindigkeit im ersten Bereich (11) reduziert wird. Hierzu wird die senkrecht zur Transportrichtung (T) verlaufende Querschnittsfläche des Kanals (5) im zweiten Bereich (12) gegenüber der senkrecht zur Transportrichtung (T) verlaufenden Querschnittsfläche aus dem ersten Bereich (11) vergrößert. Hierbei ist es vorstellbar, dass die Querschnittsfläche im zweiten Bereich (12) in vertikaler und/oder horizontaler Richtung vergrößert ist gegenüber der Querschnittsfläche im ersten Bereich (11). Dadurch lässt sich in vorteilhafter Weise eine Verweilzeit im zweiten Bereich (12) erhöhen. Weiterhin vorteilhaft könnte durch diese Maßnahme die Leistung eines Gebläses am Austritt der Vorrichtung, d.h. im Bereich der Ausgangsöffnung, reduziert werden, da bei ansonsten gleichem Volumenstrom unter Normalbedingungen der Druckverlust über die zweite Oberflächenvergrößerungsstruktur (32) verringert wird und somit das Druckniveau in der gesamten Vorrichtung (1) angehoben wird.

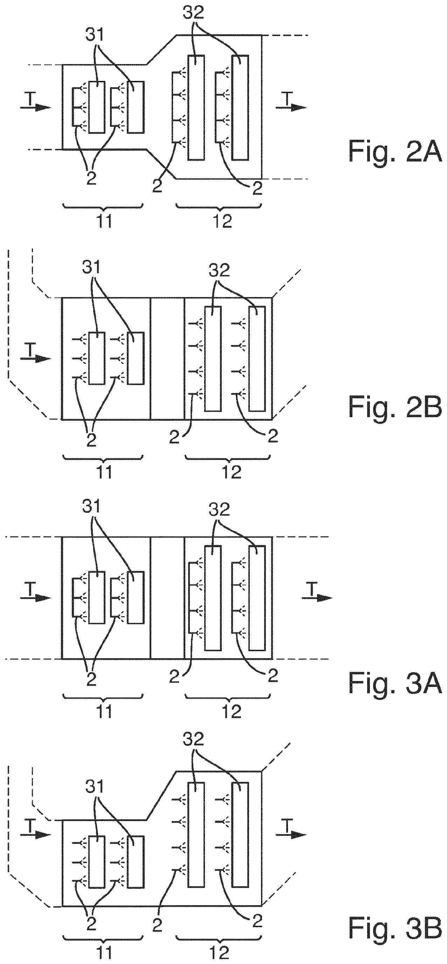

Nachfolgend wird unter Bezugnahme auf die Figuren 2A und 2B ein zweites alternatives Ausführungsbeispiel der Erfindung erläutert. Soweit die Anlagenteile mit denjenigen gemäß Figur 1 übereinstimmen, gelten die obigen Ausführungen zu dem Ausführungsbeispiel von Figur 1, so dass diese hier nicht wiederholt werden und auch die entsprechenden Bezugszeichen nicht eingezeichnet sind. Es werden hier daher nur die Unterschiede näher erläutert. Bei der Variante gemäß den Figuren 2A und 2B kann man in der Draufsicht gemäß Figur 2A erkennen, dass sich in dem zweiten Bereich (12) der Querschnitt des Kanals (5) zu beiden Seiten hin etwa im gleichen Maße erweitert, so dass sich eine zur Mittelachse des Kanals (5) symmetrische horizontale Querschnittserweiterung ergibt. Im Vergleich zu Figur 1 ist die Anordnung der Sprühvorrichtungen (2) so, dass diese jeweils etwa in Strömungsrichtung des Abgases in die Oberflächenvergrößerungsstrukturen (31, 32) hinein sprühen. Die Figuren 2A und 2B zeigen, dass jeweils sowohl nebeneinander als auch übereinander jeweils mit Abstand zueinander für jede der Oberflächenvergrößerungsstrukturen (31, 32) jeweils mehrere Sprühvorrichtungen (2) angeordnet sein können. Diese Sprühvorrichtungen (2) sind in Strömungsrichtung des Abgasstromes gesehen jeweils vor den Oberflächenvergrößerungsstrukturen (31, 32) angeordnet und sprühen in diese hinein. Figur 2B zeigt, dass hier zwar eine Querschnittserweiterung des Kanals (5) in horizontaler Richtung vorgesehen ist, die Höhe des Kanals (5), das heißt seine vertikale Ausdehnung aber über die Länge des Kanals (5) gleichbleibend ist.

Nachfolgend wird unter Bezugnahme auf die Figuren 3A und 3B ein drittes alternatives Ausführungsbeispiel der Erfindung erläutert. Soweit die Anlagenteile mit denjenigen gemäß Figur 1 übereinstimmen, gelten die obigen Ausführungen zu dem Ausführungsbeispiel von Figur 1, so dass diese hier nicht wiederholt werden und auch die entsprechenden Bezugszeichen nicht eingezeichnet sind. Es werden hier daher nur die Unterschiede näher erläutert. Bei der Variante gemäß den Figuren 3A und 3B kann man in der Draufsicht gemäß Figur 3A erkennen, dass in dem zweiten Bereich (12) der Querschnitt des Kanals (5) in der seitlichen Ausdehnung im Vergleich zu dem ersten Bereich (11) unverändert ist, jedoch ist wie Figur 3B zeigt im zweiten Bereich die Höhe des Kanals (5) größer, so dass sich eine asymmetrische vertikale Querschnittserweiterung ergibt. Im Vergleich zu Figur 1 ist die Anordnung der Sprühvorrichtungen (2) so, dass diese jeweils etwa in Strömungsrichtung des Abgases in die Oberflächenvergrößerungsstrukturen (31, 32) hinein sprühen. Die Figuren 2A und 2B zeigen, dass jeweils sowohl nebeneinander als auch übereinander jeweils mit Abstand zueinander für jede der Oberflächenvergrößerungsstrukturen (31, 32) jeweils mehrere Sprühvorrichtungen (2) angeordnet sein können. Diese Sprühvorrichtungen (2) sind in Strömungsrichtung des Abgasstromes gesehen jeweils vor den Oberflächenvergrößerungsstrukturen (31, 32) angeordnet und sprühen in diese hinein. Figur 3A zeigt, dass hier beispielsweise nur eine Querschnittserweiterung des Kanals (5) in vertikaler Richtung nach oben hin vorgesehen ist.

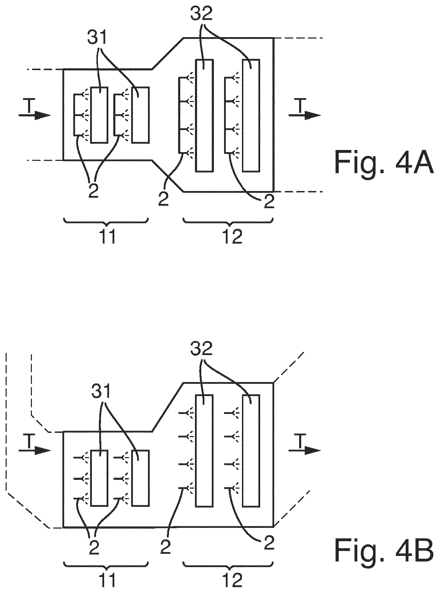

Nachfolgend wird unter Bezugnahme auf die Figuren 4A und 4B ein viertes alternatives Ausführungsbeispiel der Erfindung erläutert. Soweit die Anlagenteile mit denjenigen gemäß Figur 1 übereinstimmen, gelten die obigen Ausführungen zu dem Ausführungsbeispiel von Figur 1, so dass diese hier nicht wiederholt werden und auch die entsprechenden Bezugszeichen nicht eingezeichnet sind. Es werden hier daher nur die Unterschiede näher erläutert. Bei der Variante gemäß den Figuren 4A und 4B kann man in der Draufsicht gemäß Figur 4A erkennen, dass in dem zweiten Bereich (12) der Querschnitt des Kanals (5) in der seitlichen Ausdehnung im Vergleich zu dem ersten Bereich (11) in horizontaler Richtung zu beiden Seiten hin symmetrisch verbreitert ist und zusätzlich ist noch wie Figur 4B zeigt im zweiten Bereich (12) die Höhe des Kanals (5) größer, so dass sich zusätzlich eine asymmetrische vertikale Querschnittserweiterung ergibt. Im Vergleich zu Figur 1 ist die Anordnung der Sprühvorrichtungen (2) so, dass diese jeweils etwa in Strömungsrichtung des Abgases in die Oberflächenvergrößerungsstrukturen (31, 32) hinein sprühen. Die Figuren 4A und 4B zeigen, dass jeweils sowohl nebeneinander als auch übereinander jeweils mit Abstand zueinander für jede der Oberflächenvergrößerungsstrukturen (31, 32) jeweils mehrere Sprühvorrichtungen (2) angeordnet sein können. Diese Sprühvorrichtungen (2) sind in Strömungsrichtung des Abgasstromes gesehen jeweils vor den Oberflächenvergrößerungsstrukturen (31, 32) angeordnet und sprühen in diese hinein. Figur 4B zeigt, dass hier zusätzlich zu der horizontalen Querschnittserweiterung noch beispielsweise eine Querschnittserweiterung des Kanals (5) in vertikaler Richtung nach oben hin vorgesehen ist.

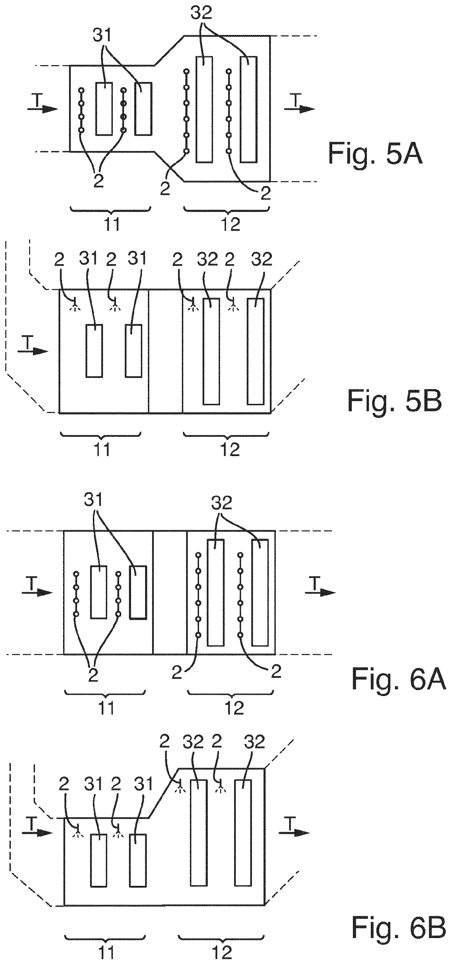

Nachfolgend wird unter Bezugnahme auf die Figuren 5A und 5B ein fünftes alternatives Ausführungsbeispiel der Erfindung erläutert. Soweit die Anlagenteile mit denjenigen gemäß Figur 1 übereinstimmen, gelten die obigen Ausführungen zu dem Ausführungsbeispiel von Figur 1, so dass diese hier nicht wiederholt werden und auch die entsprechenden Bezugszeichen nicht eingezeichnet sind. Es werden hier daher nur die Unterschiede näher erläutert. Bei der Variante gemäß den Figuren 5A und 5B kann man in der Draufsicht gemäß Figur 5A erkennen, dass sich in dem zweiten Bereich (12) der Querschnitt des Kanals (5) zu beiden Seiten hin etwa im gleichen Maße erweitert, so dass sich eine zur Mittelachse des Kanals (5) symmetrische horizontale Querschnittserweiterung ergibt. Im Vergleich zu Figur 2 ist die Anordnung der Sprühvorrichtungen (2) jedoch hier so, dass diese jeweils etwa von oben her etwa rechtwinklig (also quer) zur Strömungsrichtung des Abgases in den Abgasstrom hinein sprühen und zwar stromaufwärts vor den Oberflächenvergrößerungsstrukturen (31, 32), so dass das Sprühmedium von dem Abgasstrom mitgerissen wird. Die Figuren 5A und 5B zeigen, dass mehrere jeweils nebeneinander, mit Abstand zueinander in Reihen angeordnete Sprühvorrichtungen (2) für jede der Oberflächenvergrößerungsstrukturen (31, 32) angeordnet sein können. Diese Sprühvorrichtungen (2) sind in Strömungsrichtung des Abgasstromes gesehen jeweils vor den Oberflächenvergrößerungsstrukturen (31, 32) angeordnet und sprühen vor diese und nach unten hin. Figur 5B zeigt, dass hier zwar eine Querschnittserweiterung des Kanals (5) in horizontaler Richtung vorgesehen ist, die Höhe des Kanals (5), das heißt seine vertikale Ausdehnung aber über die Länge des Kanals (5) gleichbleibend ist.

Nachfolgend wird unter Bezugnahme auf die Figuren 6A und 6B ein sechstes alternatives Ausführungsbeispiel der Erfindung erläutert. Soweit die Anlagenteile mit denjenigen gemäß Figur 1 übereinstimmen, gelten die obigen Ausführungen zu dem Ausführungsbeispiel von Figur 1, so dass diese hier nicht wiederholt werden und auch die entsprechenden Bezugszeichen nicht eingezeichnet sind. Es werden hier daher nur die Unterschiede näher erläutert. Bei der Variante gemäß den Figuren 6A und 6B kann man in der Draufsicht gemäß Figur 6A erkennen, dass in dem zweiten Bereich (12) der Querschnitt des Kanals (5) in der seitlichen Ausdehnung im Vergleich zu dem ersten Bereich (11) unverändert ist, jedoch ist wie Figur 6B zeigt im zweiten Bereich die Höhe des Kanals (5) größer, so dass sich eine asymmetrische vertikale Querschnittserweiterung ergibt. Im Vergleich zu Figur 1 ist die Anordnung der Sprühvorrichtungen (2) so, dass diese jeweils quer zur Strömungsrichtung des Abgases vor die Oberflächenvergrößerungsstrukturen (31, 32) in den Abgasstrom hinein sprühen. Die Figuren 6A und 6B zeigen, dass jeweils nebeneinander, jeweils mit Abstand zueinander für jede der Oberflächenvergrößerungsstrukturen (31, 32) jeweils mehrere Sprühvorrichtungen (2) angeordnet sein können. Diese Sprühvorrichtungen (2) sind in Strömungsrichtung des Abgasstromes gesehen jeweils vor den Oberflächenvergrößerungsstrukturen (31, 32) angeordnet und sprühen vom oberen Bereich des Kanals her vor die Oberflächenvergrößerungsstrukturen (31, 32). Figur 6A zeigt, dass hier beispielsweise nur eine Querschnittserweiterung des Kanals (5) in vertikaler Richtung nach oben hin vorgesehen ist.

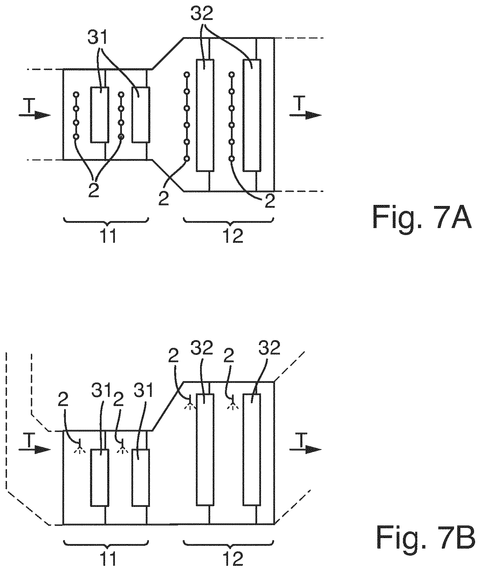

Nachfolgend wird unter Bezugnahme auf die Figuren 7A und 7B ein siebtes alternatives Ausführungsbeispiel der Erfindung erläutert. Soweit die Anlagenteile mit denjenigen gemäß Figur 1 übereinstimmen, gelten die obigen Ausführungen zu dem Ausführungsbeispiel von Figur 1, so dass diese hier nicht wiederholt werden und auch die entsprechenden Bezugszeichen nicht eingezeichnet sind. Es werden hier daher nur die Unterschiede näher erläutert. Bei der Variante gemäß den Figuren 7A und 7B kann man in der Draufsicht gemäß Figur 7A erkennen, dass in dem zweiten Bereich (12) der Querschnitt des Kanals (5) in der seitlichen Ausdehnung im Vergleich zu dem ersten Bereich (11) in horizontaler Richtung zu beiden Seiten hin symmetrisch verbreitert ist und zusätzlich ist noch wie Figur 7B zeigt im zweiten Bereich (12) die Höhe des Kanals (5) größer, so dass sich zusätzlich eine asymmetrische vertikale Querschnittserweiterung ergibt. Im Vergleich zu Figur 1 ist die Anordnung der Sprühvorrichtungen (2) so, dass diese jeweils von oben her quer zur Strömungsrichtung des Abgases vor die Oberflächenvergrößerungsstrukturen (31, 32) und in den Abgasstrom hinein sprühen. Die Figuren 7A und 7B zeigen, dass jeweils nebeneinander, jeweils mit Abstand zueinander in Reihen angeordnet für jede der Oberflächenvergrößerungsstrukturen (31, 32) jeweils mehrere Sprühvorrichtungen (2) angeordnet sein können, wobei die Sprühvorrichtungen (2) in dem im Querschnitt erweiterten zweiten Bereich (12) aufgrund der größeren Höhe des Kanals (5) weiter oben angeordnet sein können als die Sprühvorrichtungen (2) in dem ersten Bereich (11). Diese Sprühvorrichtungen (2) sind in Strömungsrichtung des Abgasstromes gesehen jeweils vor den Oberflächenvergrößerungsstrukturen (31, 32) angeordnet und sprühen von oben her stromaufwärts vor diesen in den Abgasstrom hinein. Figur 7B zeigt, dass hier zusätzlich zu der horizontalen Querschnittserweiterung noch beispielsweise eine Querschnittserweiterung des Kanals (5) in vertikaler Richtung nach oben hin vorgesehen ist.

### Experimentelle Daten

Zum Nachweis der Abhängigkeit des Wirkungsgrads für die Ammoniakentfernung aus dem Abgas von der Geschwindigkeit des Gases wurde folgender Versuch durchgeführt:
Mit Ammoniak angereichte Luft wurde in einen Kanal mit Sprühvorrichtungen (2) und einer zweite Oberflächenvergrößerungsstruktur (32), wie sie für den zweiten Bereich (12) des Kanals aus dem vorangegangenen Ausführungsbeispiel vorgesehen sind, eingeleitet. Dabei wurde die Geschwindigkeit des Abgases innerhalb des Kanals (5) mittels eines Ventilators reguliert bzw. eingestellt. Zur quantitativen Bestimmung des Wirkungsgrads wurde der Ammoniakgehalt vor Eintritt in den Kanal (5) und bei Austritt aus dem Kanal (5) gemessen und die Ammoniakabnahme gemäß 1-(NH3,ₒᵤₜ/NH3_{,in}) bestimmt. Zur Bestimmung der jeweiligen Ammoniakkonzentrationen wurde eine entsprechende Teilgasmenge aus dem zu untersuchenden Gas abgezogen und durch eine genau bekannte Menge von Schwefelsäure geleitet. Die Teilgasmenge wird durch geeignete Einrichtungen (Gaszähler) genau bestimmt. Durch die Reaktion des Ammoniaks mit der Schwefelsäure entsteht Ammoniumsulfat, welches den Anteil an freier Schwefelsäure reduziert. Nach Beendigung des Durchleitens wird durch Rücktitration mit Lauge (Natronlauge) auf dem Fachmann geläufige Art die Restmenge an noch vorhandener Schwefelsäure ermittelt. Mit den so erhaltenden Daten kann die Ammoniakkonzentration in dem Gasstrom berechnet werden.

Das Ergebnis der Messung war eine Steigerung der Abnahme des Ammoniak-Gehaltes um 2,7%-Punkte (was einer Steigerung des Wirkungsgrades von 3,1 % entspricht), wenn die Gasgeschwindigkeit im Kanal (5) um 10 % reduziert wurde.

### Bezugszeichenliste

- 1: Vorrichtung
- 2: Sprühvorrichtung
- 5: Kanal
- 10: Eingangsöffnung
- 11: erster Bereich
- 12: zweiter Bereich
- 20: Ausgangsöffnung
- 21: erstes Lösungsmittel
- 22: zweites Lösungsmittel
- 31: Oberflächenvergrößerungsstruktur
- 32: Oberflächenvergrößerungsstruktur
- T: Transportrichtung (T)

## Patentansprüche

1. Harnstoffanlage, in die eine Vorrichtung (1) zur Abgaswäsche integriert ist, wobei die Vorrichtung einen Kanal (5) mit einer Eingangsöffnung (10) und einer Ausgangsöffnung (20) umfasst, wobei die Vorrichtung (1) derart ausgestaltet ist, dass ein Abgas entlang einer Transportrichtung (T) im Kanal (5)
- einen ersten Bereich (11) zur Entfernung von Staubpartikeln aus dem Abgas und
- einen zweiten Bereich (12) zur Entfernung von chemischen Verbindungen aus dem Abgas, welche durch eine Säure-Base-Reaktion in eine wässrige Flüssigphase integriert werden können, passiert,
wobei eine senkrecht zur Transportrichtung (T) verlaufende Querschnittsfläche des Kanals (5) im zweiten Bereich (12) größer ist als eine senkrecht zur Transportrichtung (T) verlaufende Querschnittsfläche im ersten Bereich (11),
**dadurch gekennzeichnet, dass** die Vorrichtung (1) derart ausgestaltet ist, dass der Kanal (5) im verbauten Zustand der Vorrichtung (1), zumindest abschnittsweise horizontal verläuft und/oder die Transportrichtung (T) des Abgases durch den Kanal (5) horizontal verläuft, wobei der erste Bereich (11) eine in dem Kanal (5) angeordnete erste Oberflächenvergrößerungsstruktur (31) zur Entfernung von Harnstoffstaubpartikeln, und der zweite Bereich eine in dem Kanal (5) angeordnete zweite Oberflächenvergrößerungsstruktur (32) zur Entfernung von chemischen Verbindungen aus dem Abgas, welche durch eine Säure-Base-Reaktion in eine wässrige Flüssigphase integriert werden können, insbesondere von Ammoniak, umfasst.

2. Vorrichtung (1) gemäß Anspruch 1, **dadurch gekennzeichnet, dass** die größere Querschnittsfläche in dem zweiten Bereich (12) sich dadurch ergibt, dass dieser zweite Bereich (12) eine größere Höhe hat als der erste Bereich (11) und/oder dadurch, dass der zweite Bereich (12) eine größere Breite hat als der erste Bereich (11).

3. Vorrichtung (1) gemäß einem der vorhergehenden Ansprüche, **dadurch gekennzeichnet, dass** der erste Bereich (11) wenigstens eine Sprühvorrichtung (2) zum Versprühen eines ersten Lösungsmittels (21) und der zweite Bereich (12) wenigstens eine weitere Sprühvorrichtung (2) zum Versprühen eines zweiten Lösungsmittels (22) umfasst.

4. Vorrichtung (1) gemäß Anspruch 3, **dadurch gekennzeichnet, dass** die Sprühvorrichtung (2) und/oder die Sprühvorrichtungen des ersten Bereichs (11) und/oder des zweiten Bereichs (12) ein Verteilerrohr umfasst.

5. Vorrichtung (1) gemäß Anspruch 4, **dadurch gekennzeichnet, dass** das Verteilerrohr der Sprühvorrichtung (2) des ersten Bereichs (11) und/oder des zweiten Bereichs (12) mehrere Sprühdüsen umfasst, wobei die Sprühdüsen im ersten Bereich (11) und/oder im zweiten Bereich (12) jeweils über eine gemeinsame Zuleitung mit Lösungsmittel (21, 22) versorgt werden.

6. Vorrichtung (1) gemäß Anspruch 1, **dadurch gekennzeichnet, dass** die Sprühvorrichtung (2) oder die Sprühvorrichtungen (2) jeweils derart angeordnet sind, dass sie von oben her quer zur Strömungsrichtung des Abgasstromes stromaufwärts vor den Oberflächenvergrößerungsstrukturen (31, 32) in den Abgasstrom hinein sprühen und/oder dass die Sprühvorrichtungen (2) jeweils stromaufwärts vor den Oberflächenvergrößerungsstrukturen (31, 32) angeordnet sind, derart, dass sie in Strömungsrichtung des Abgasstromes in diesen hinein sprühen.

7. Vorrichtung (1) gemäß Anspruch 1 oder 6, **dadurch gekennzeichnet, dass** die erste Oberflächenvergrößerungsstruktur (31) und/oder die zweite Oberflächenvergrößerungsstruktur (32)
- regellose Strukturelemente ausgewählt aus der Gruppe bestehend aus Raschig-Ringen, Pall-Ringen, Sattel-Körpern, Feder-typischen Körpern und Kombinationen davon; und/oder
- strukturierte Strukturelemente ausgewählt aus der Gruppe bestehend aus Blechen, gewellten Blechen, geformten Blechen und Kombinationen davon; und/oder
Materialien ausgewählt aus der Gruppe bestehend aus Metall, Glas, Kunststoff, Carbonfasern und Kombinationen davon.

8. Vorrichtung (1) gemäß einem der vorhergehenden Ansprüche, **dadurch gekennzeichnet, dass** der erste Bereich (11) zur Entfernung von Staubpartikeln, insbesondere von Harnstoffstaubpartikeln, mehrstufig ausgestaltet ist und/oder der zweite Bereich (12) zur Entfernung von chemischen Verbindungen aus dem Abgas, welche durch eine Säure-Base-Reaktion in eine wässrige Flüssigphase integriert werden können, insbesondere von Ammoniak, ein-
oder mehrstufig ausgestaltet ist.

9. Verfahren zur Abgaswäsche, wobei ein Abgas durch einen Kanal (5) geführt wird, umfassend einen ersten Bereich (11) zur Entfernung von Harnstoffstaubpartikeln und umfassend einen zweiten Bereich (12) zur Entfernung von chemischen Verbindungen aus dem Abgas, welche durch eine Säure-Base-Reaktion in eine wässrige Flüssigphase integriert werden können, insbesondere von Ammoniak, wobei durch eine Vergrößerung einer senkrecht zur Transportrichtung (T) verlaufenden Querschnittsfläche des Kanals (5) im zweiten Bereich (12) eine geringere Abgasgeschwindigkeit, mit der das Abgas im Kanal (5) geführt wird, als im ersten Bereich (11) vorliegt, **dadurch gekennzeichnet, dass** der Kanal (5) zumindest abschnittsweise horizontal verläuft und/oder die Transportrichtung (T) des Abgases durch den Kanal (5) horizontal verläuft, wobei das Verfahren in einer Harnstoffanlage stattfindet.

10. Verfahren gemäß Anspruch 9, wobei im zweiten Bereich (12) Ammoniak mittels einer sauren Waschlösung entfernt wird.

11. Verfahren nach Anspruch 10, **dadurch gekennzeichnet, dass** die saure Waschlösung eine Säure enthält, insbesondere Schwefelsäure, Salpetersäure, Salzsäure und/oder Phosphorsäure.

12. Verfahren gemäß einem oder mehreren der Ansprüche 9 bis 11, wobei das Abgas mit einer Geschwindigkeit von mehr als 1 m/s, vorzugsweise mehr als 5 m/s und besonders bevorzugt mehr als 10 m/s durch den ersten Bereich (11) transportiert wird; und/oder das Abgas mit einer Geschwindigkeit von weniger als 10 m/s, vorzugsweise weniger als 5 m/s und besonders bevorzugt weniger als 1 m/s durch den zweiten Bereich (12) transportiert wird; und/oder das durch Eingangsöffnung (10) eintretende Abgas eine Temperatur von maximal 140 °C, bevorzugt maximal 120 °C, bevorzugt maximal 100 °C, bevorzugt maximal 80 °C, bevorzugt maximal 60 °C, oder bevorzugt maximal 50 °C aufweist.

13. Verwendung einer Harnstoffanlage gemäß einem oder mehreren der Ansprüche 1 bis 8 in einem Verfahren zur Abgaswäsche gemäß einem der Ansprüche 9 bis 12.

## Claims

1. A urea plant, into which a device (1) for waste gas scrubbing is integrated, wherein the device comprises a duct (5) having an inlet opening (10) and an outlet opening (20), wherein the device (1) is designed in such a way that a waste gas passes, along a transportation direction (T) in the duct (5),
- a first region (11) for removing dust particles from the waste gas and
- a second region (12) for removing chemical compounds from the waste gas, which can be integrated by an acid-base reaction into an aqueous liquid phase,
wherein a cross-sectional area of the duct (5) extending perpendicularly to the transportation direction (T) in the second region (12) is greater than a cross-sectional area extending perpendicularly to the transportation direction (T) in the first region (11), **characterized in that** the device (1) is designed in such a way that the duct (5) extends horizontally at least in sections and/or the transportation direction (T) of the waste gas through the duct (5) extends horizontally in the installed state of the device (1), wherein the first region (11) comprises a first surface enlargement structure (31) arranged in the duct (5) for removing urea dust particles, and the second region comprises a second surface enlargement structure (32) arranged in the duct (5) for removing chemical compounds from the waste gas, which can be integrated by an acid-base reaction into an aqueous liquid phase, in particular of ammonia.

2. The device (1) as claimed in claim 1, **characterized in that** the larger cross-sectional area in the second region (12) results because this second region (12) has a greater height than the first region (11) and/or because the second region (12) has a greater width than the first region (11).

3. The device (1) as claimed in either one of the preceding claims, **characterized in that** the first region (11) comprises at least one spraying device (2) for spraying a first solvent (21) and the second region (12) comprises at least one further spraying device (2) for spraying a second solvent (22).

4. The device (1) as claimed in claim 3, **characterized in that** the spraying device (2) and/or the spraying devices of the first region (11) and/or the second region (12) comprises a distributor pipe.

5. The device (1) as claimed in claim 4, **characterized in that** the distributor pipe of the spraying device (2) of the first region (11) and/or the second region (12) comprises multiple spray nozzles, wherein the spray nozzles in the first region (11) and/or in the second region (12) are each supplied with solvent (21, 22) via a common feed line.

6. The device (1) as claimed in claim 1, **characterized in that** the spraying device (2) or the spraying devices (2) are each arranged in such a way that they spray into the waste gas flow from above transversely to the flow direction of the waste gas flow upstream in front of the surface enlargement structures (31, 32) and/or the spraying devices (2) are each arranged upstream in front of the surface enlargement structures (31, 32) in such a way that they spray into these structures in the flow direction of the waste gas flow.

7. The device (1) as claimed in claim 1 or 6, **characterized in that** the first surface enlargement structure (31) and/or the second surface enlargement structure (32) comprises
- random structural elements selected from the group consisting of Raschig rings, Pall rings, saddle elements, spring-typical bodies, and combinations thereof; and/or
- structured structural elements selected from the group consisting of plates, corrugated plates, formed plates, and combinations thereof; and/or
- material selected from the group consisting of metal, glass, plastic, carbon fibers, and combinations thereof.

8. The device (1) as claimed in any one of the preceding claims, **characterized in that** the first region (11) is designed for removing dust particles, in particular urea dust particles, in multiple steps, and/or the second region (12) is designed for removing chemical compounds from the waste gas, which can be integrated into an aqueous liquid phase by an acid-base reaction, in particular ammonia, in one or multiple steps.

9. A method for waste gas scrubbing, wherein a waste gas is guided through a duct (5), comprising a first region (11) for removing urea dust particles, and comprising a second region (12) for removing chemical compounds from the waste gas, which can be integrated by an acid-base reaction into an aqueous liquid phase, in particular of ammonia, wherein due to an enlargement of a cross-sectional area of the duct (5) extending perpendicularly to the transportation direction (T) in the second region (12), a lower waste gas speed at which the waste gas is guided in the duct (5) is provided than in the first region (11), **characterized in that** the duct (5) extends horizontally at least in sections and/or the transportation direction (T) of the waste gas through the duct (5) extends horizontally, wherein the method takes place in a urea plant.

10. The method as claimed in claim 9, wherein ammonia is removed by means of an acid washing solution in the second region (12).

11. The method as claimed in claim 10, **characterized in that** the acid washing solution contains an acid, in particular sulfuric acid, nitric acid, hydrochloric acid, and/or phosphoric acid.

12. The method as claimed in one or more of claims 9 to 11, wherein the waste gas is transported at a speed of greater than 1 m/s, preferably greater than 5 m/s, and particularly preferably greater than 10 m/s through the first region (11); and/or the waste gas is transported at a speed of less than 10 m/s, preferably less than 5 m/s, and particularly preferably less than 1 m/s through the second region (12); and/or the waste gas entering through the inlet opening (10) has a temperature of at most 140°C, preferably at most 120°C, preferably at most 100°C, preferably at most 80°C, preferably at most 60°C, or preferably at most 50°C.

13. A use of a urea plant as claimed in one or more of claims 1 to 8 in a method for waste gas scrubbing as claimed in any one of claims 9 to 12.

## Revendications

1. Unité de production d'urée, dans laquelle un dispositif (1) pour l'épuration de gaz d'échappement est intégré, le dispositif comprenant un canal (5) muni d'une ouverture d'entrée (10) et d'une ouverture de sortie (20), le dispositif (1) étant configuré de telle sorte qu'un gaz d'échappement traverse le long d'une direction de transport (T) dans le canal (5)
- une première zone (11) pour l'élimination de particules de poussière du gaz d'échappement, et
- une deuxième zone (12) pour l'élimination de composés chimiques du gaz d'échappement, qui peuvent être intégrés par une réaction acide-base dans une phase liquide aqueuse,
une surface de section transversale, perpendiculaire à la direction de transport (T), du canal (5) dans la deuxième zone (12) étant plus grande qu'une surface de section transversale, perpendiculaire à la direction de transport (T), dans la première zone (11),
**caractérisée en ce que** le dispositif (1) est configuré de telle sorte que le canal (5), à l'état monté du dispositif (1), soit horizontal au moins en sections et/ou que la direction de transport (T) du gaz d'échappement au travers du canal (5) soit horizontale, la première zone (11) comprenant une première structure d'agrandissement de surface (31) agencée dans le canal (5) pour l'élimination de particules de poussière d'urée, et la deuxième zone comprenant une deuxième structure d'agrandissement de surface (32) agencée dans le canal (5) pour l'élimination de composés chimiques du gaz d'échappement, qui peuvent être intégrés par une réaction acide-base dans une phase liquide aqueuse, notamment d'ammoniac.

2. Dispositif (1) selon la revendication 1,
**caractérisé en ce que** la surface de section transversale plus grande dans la deuxième zone (12) est obtenue **en ce que** cette deuxième zone (12) a une hauteur plus grande que la première zone (11) et/ou **en ce que** la deuxième zone (12) a une largeur plus grande que la première zone (11).

3. Dispositif (1) selon l'une quelconque des revendications précédentes, **caractérisé en ce que** la première zone (11) comprend au moins un dispositif de pulvérisation (2) pour la pulvérisation d'un premier solvant (21) et la deuxième zone (12) comprend au moins un dispositif de pulvérisation supplémentaire (2) pour la pulvérisation d'un deuxième solvant (22).

4. Dispositif (1) selon la revendication 3,
**caractérisé en ce que** le dispositif de pulvérisation (2) et/ou les dispositifs de pulvérisation de la première zone (11) et/ou de la deuxième zone (12) comprennent un tube de distribution.

5. Dispositif (1) selon la revendication 4,
**caractérisé en ce que** le tube de distribution du dispositif de pulvérisation (2) de la première zone (11) et/ou de la deuxième zone (12) comprend plusieurs buses de pulvérisation, les buses de pulvérisation dans la première zone (11) et/ou dans la deuxième zone (12) étant respectivement alimentées par l'intermédiaire d'une conduite d'alimentation commune avec du solvant (21, 22).

6. Dispositif (1) selon la revendication 1,
**caractérisé en ce que** le dispositif de pulvérisation (2) ou les dispositifs de pulvérisation (2) sont respectivement agencés de telle sorte qu'ils pulvérisent depuis le haut perpendiculairement à la direction d'écoulement du courant de gaz d'échappement en amont avant les structures d'agrandissement de surface (31, 32) dans le courant de gaz d'échappement et/ou **en ce que** les dispositifs de pulvérisation (2) sont respectivement agencés en amont avant les structures d'agrandissement de surface (31, 32), de telle sorte qu'ils pulvérisent dans la direction d'écoulement du courant de gaz d'échappement dans celui-ci.

7. Dispositif (1) selon la revendication 1 ou 6, **caractérisé en ce que** la première structure d'agrandissement de surface (31) et/ou la deuxième structure d'agrandissement de surface (32) comprennent
- des éléments structuraux irréguliers choisis dans le groupe constitué par les anneaux de Raschig, les anneaux de Pall, les selles de Berl, les corps de type ressort et des combinaisons de ceux-ci ; et/ou
- des éléments structuraux structurés choisis dans le groupe constitué par les tôles, les tôles ondulées, les tôles façonnées et des combinaisons de ceux-ci ; et/ou
des matériaux choisis dans le groupe constitué par le métal, le verre, le plastique, les fibres de carbone et des combinaisons de ceux-ci.

8. Dispositif (1) selon l'une quelconque des revendications précédentes, **caractérisé en ce que** la première zone (11) pour l'élimination de particules de poussière, notamment de particules de poussière d'urée, est configurée à plusieurs niveaux, et/ou la deuxième zone (12) pour l'élimination de composés chimiques du gaz d'échappement, qui peuvent être intégrés par une réaction acide-base dans une phase liquide aqueuse, notamment d'ammoniac, est configurée à un ou plusieurs niveaux.

9. Procédé d'épuration de gaz d'échappement, dans lequel un gaz d'échappement est acheminé au travers d'un canal (5), comprenant une première zone (11) pour l'élimination de particules de poussière d'urée et comprenant une deuxième zone (12) pour l'élimination de composés chimiques du gaz d'échappement, qui peuvent être intégrés par une réaction acide-base dans une phase liquide aqueuse, notamment d'ammoniac, dans lequel, par un agrandissement d'une surface de section transversale, perpendiculaire à la direction de transport (T), du canal (5) dans la deuxième zone (12), une vitesse de gaz d'échappement plus faible, avec laquelle le gaz d'échappement est acheminé dans le canal (5), que dans la première zone (11) est présente, **caractérisé en ce que** le canal (5) est horizontal au moins en sections et/ou la direction de transport (T) du gaz d'échappement au travers du canal (5) est horizontale, le procédé ayant lieu dans une unité de production d'urée.

10. Procédé selon la revendication 9, dans lequel de l'ammoniac est éliminé au moyen d'une solution d'épuration acide dans la deuxième zone (12).

11. Procédé selon la revendication 10, **caractérisé en ce que** la solution de lavage acide contient un acide, notamment de l'acide sulfurique, de l'acide nitrique, de l'acide chlorhydrique et/ou de l'acide phosphorique.

12. Procédé selon une ou plusieurs des revendications 9 à 11, dans lequel le gaz d'échappement est transporté avec une vitesse de plus de 1 m/s, de préférence de plus de 5 m/s et de manière particulièrement préférée de plus de 10 m/s, au travers de la première zone (11) ; et/ou le gaz d'échappement est transporté avec une vitesse de moins de 10 m/s, de préférence de moins de 5 m/s et de manière particulièrement préférée de moins de 1 m/s, au travers de la deuxième zone (12) ; et/ou le gaz d'échappement entrant par l'ouverture d'entrée (10) présente une température d'au plus 140 °C, de préférence d'au plus 120 °C, de préférence d'au plus 100 °C, de préférence d'au plus 80 °C, de préférence d'au plus 60 °C, ou de préférence d'au plus 50 °C.

13. Utilisation d'une unité de production d'urée selon une ou plusieurs des revendications 1 à 8 dans un procédé pour l'épuration de gaz d'échappement selon l'une quelconque des revendications 9 à 12.
